# EUROPÄISCHE PATENTSCHRIFT

(11) **EP 0 761 200 B1**
(45) Veröffentlichungstag und Bekanntmachung des Hinweises auf die Patenterteilung: **18.04.2001**
(21) Anmeldenummer: 96112868.3
(22) Anmeldetag: 09.08.1996
(51) Int. Cl.: A61K 7/13, A61K 7/06

(54) **Mittel zur Färbung und Tönung von menschlichen Haaren**
Composition for dyeing and tinting of human hair
Composition pour colorer ou teindre les cheveux humains

(30) Priorität: 23.08.1995 DE 19530998
(43) Veröffentlichungstag der Anmeldung: 12.03.1997
(73) Patentinhaber: GOLDWELL GmbH, 64297 Darmstadt (DE)
(72) Erfinder: Eberling, Walter, 64560 Riedstadt-Crumstadt (DE); Lorenz, Heribert, 64401 Gross-Bieberau (DE); Klusch, Hans, 64319 Pfungstadt (DE); Petzke, Erika, 64319 Pfungstadt (DE)

(56) Entgegenhaltungen:
- WO-A-96/18376
- DE-A- 4 233 874
- GB-A- 2 254 341

## Beschreibung

Die vorliegende Erfindung betrifft ein pulverförmiges Mittel zum Färben bzw. Tönen von menschlichen Haaren mit erhöhter Färbeintensität und verbesserten Gebrauchseigenschaften.

Es ist allgemein bekannt, daß Haarfärbemittel in zwei Kategorien aufgeteilt werden, nämlich einerseits die permanenten Haarfärbemittel, die grundsätzlich Haarfarbstoffvorprodukte enthalten, die zusammen mit Oxidationsmitteln je nach Zusammensetzung die gewünschte Färbung auf dem Haar entwickeln; und andererseits semipermanente Haarfärbemittel, die direktziehende Farbstoffe enthalten, die zur Entwicklung ihrer Färbeleistung keinerlei Oxidationsmittelzusatzes bedürfen. Dementsprechend sind die Färbungen auch weniger dauerhaft als diejenigen mit Permanentfarbstoffen erzielbaren.

Diese Farbzusammensetzungen auf Basis direktziehender Farbstoffe werden in der Regel entweder als Tönungsshampoos, als -lotionen oder als Tönungsfestiger, gegebenenfalls auch als Aerosolschaum, appliziert. Vereinzelt wurde, insbesondere für Färbemittel auf Basis von pflanzlichen Farbstoffen, auch bereits die Verwendung von pulverförmigen Produkten vorgeschlagen, die vor ihrer Anwendung auf dem Haar mit Wasser angerührt werden müssen.

Dabei ergeben sich jedoch häufig Gebrauchsprobleme, da die so erzielten Färbezusammensetzungen verklumpen und keine präzise, gleichmäßige Applikation zulassen. Darüber hinaus ist die mit diesen Zusammensetzungen erzielbare Farbintensität ebenfalls nicht befriedigend.

Es bestand daher ein Bedürfnis nach pulverförmigen Haarfärbeprodukten auf Basis direktziehender Farbstoffe, die eine problemlose Anwendung gestatten und eine gegenüber üblichen Zusammensetzungen verbesserte Färbewirkung aufweisen.

Erfindungsgemäß wird nunmehr ein pulverförmiges Mittel zum Färben und Tönen von menschlichen Haaren zur Verfügung gestellt, das mindestens einen direktziehenden Farbstoff sowie zusätzlich 1 bis 50, vorzugsweise 2,5 bis 35, insbesondere 5 bis 25 Gew.-%, berechnet auf die Gesamtzusammensetzung, mindestens eines Hydroxy-C₂-C₄-alkyl Guar Gums bzw. dessen quaternären Salzen enthält.

Durch den Zusatz dieser Guar Gum-Derivate wird die Färbeintensität, d.h. das Farbaufziehvermögen, der Zusammensetzung gegenüber herkömmlichen Produkten wesentlich erhöht; das Pulver läßt sich darüber hinaus mit Wasser ausgezeichnet zu stabilen, keine Verklumpungen aufweisenden, auf das Haar auftragefähigen flüssigen Zubereitungen anrühren.

Bevorzugte Guar Gum-Derivate sind Hydroxypropyl-Guar, d.h., der Propylenglycolether des Guar Gums, sowie auch Quaternisierungsprodukte desselben, insbesondere das Hydroxypropyl-Guar-hdroxypropyltrimoniumchlorid. Weiter geeignete Hydroxyalkyl-Guar-Derivate sind beispielsweise Hydroxyethyl-Guar, Hydroxybutyl-Guar und deren Quaternisierungsprodukte.

Geeignete Handelsprodukte sind unter den Markennamen "Jaguar HP ®", "Jaguar C-17 ®" und "Jaguar C-162 ®" auf dem Markt.

Als direktziehende Haarfarbstoffe können im Prinzip alle für diesen Zweck zugelassenen Farbstoffe verwendet werden; es wird hierzu insbesondere auf die deutsche "Verordnung über kosmetische Mittel (Kosmetik-VO)" in der jeweils geltenden Fassung, Anlage 3, verwiesen.

Bevorzugt sind jedoch die kationischen (basischen) Farbstoffe, da deren Stabilität und Farbaufziehvermögen durch den Zusatz der erfindungsgemäßen Galactomannan-Derivate besonders erhöht wird.

Aus der DE-A 42 33 874 sind granulierte Haarfärbemittel bekannt, die als Bindemittel Guarmehl enthalten.

Besonders geeignete basische (kationische) Farbstoffe sind:
- Basic Blue 6,: C.I.-No. 51,175;
- Basic Blue 7,: C.I.-No. 42,595;
- Basic Blue 9,: C.I.-No. 52,015;
- Basic Blue 26,: C.I.-No. 44,045;
- Basic Blue 41,: C.I.-No. 11,154;
- Basic Blue 99,: C.I.-No. 56,059;
- Basic Brown 4,: C.I.-No. 21,010;
- Basic Brown 16,: C.I.-No. 12,250;
- Basic Brown 17,: C.I.-No. 12,251;
- Basic Green 1,: C.I.-No. 42,040;
- Basic Red 2,: C.I.-No. 50,240;
- Basic Red 22,: C.I.-No. 11,055;
- Basic Red 76,: C.I.-No. 12,245;
- Basic Violet 1,: C.I.-No. 42,535;
- Basic Violet 3,: C.I.-No. 42,555;
- Basic Violet 10,: C.I.-No. 45,170;
- Basic Violet 14,: C.I.-No. 42,510;
- Basic Yellow 57,: C.I.-No. 12,719

Als mögliche saure (anionische) Farbstoffe können Verwendung finden:
- Acid Black 1,: C.I.-No. 20,470;
- Acid Blue 9,: C.I.-No. 42,090;
- Acid Blue 74,: C.I.-No. 73,015;
- Acid Red 18,: C.I.-No. 16,255;
- Acid Red 27,: C.I.-No. 16,185;
- Acid Red 87,: C.I.-No. 45,380;
- Acid Red 92,: C.I.-No. 45,410;
- Acid Violet 43,: C.I.-No. 60,730;
- Acid Yellow 1,: C.I.-No. 10,316;
- Acid Yellow 23,: C.I.-No. 19,140;
- Acid Yellow 3,: C.I.-No. 47,005;
- D&C Brown No.1,: C.I.-No. 20,170;
- D&C Green No.5,: C.I.-No. 61,570;
- D&C Orange No.4,: C.I.-No. 15,510;
- D&C Orange No.10,: C.I.-No. 45,425:1;
- D&C Orange No.11,: C.I.-No. 45,425;
- D&C Red No.21,: C.I.-No. 45,380:2;
- D&C Red No.27,: C.I.-No. 45,410:1;
- D&C Red No.33,: C.I.-No. 17,200;
- D&C Yellow No.7,: C.I.-No. 45,350:1;
- D&C Yellow No.8,: C.I.-No. 45,350;
- FD&C Red No.4,: C.I.-No. 14,700;
- FD&C Yellow No.6,: C.I.-No. 15,985.
Auch pflanzliche Farbstoffe können allein oder in Kombination mit synthetischen Direktziehern Verwendung finden, beispielsweise Henna (rot oder schwarz), Alkannawurzel, Laccainsäure (Stocklack), Blauholzpulver, Krappwurzel- und Rhabarberwurzelpulver, etc.

Der Anteil der direktziehenden Farbstoffe in den erfindungsgemäßen pulverförmigen Zusammensetzungen ist je nach gewünschtem Farbton variabel und liegt im allgemeinen zwischen etwa 0,1 bis etwa 25, vorzugsweise 0,5 bis 10, insbesondere 1 bis 5 Gew.-% des Mittels.

Das Haarfärbe- und Tönungsmittel nach der Erfindung kann mindestens ein synthetisches oder natürliches haarkonditionierendes Polymeres, vorzugsweise in einer Menge von 0,1 bis 10, insbesondere 0,5 bis 5 Gew.-% der Gesamtzusammensetzung, enthalten. Obwohl grundsätzlich alle Arten von Polymeren verwendet werden können, also nichtionische, anionische, amphotere und kationische Polymere, werden kationische Polymere im Rahmen der Erfindung bevorzugt.

Die erfindungsgemäßen Farbpulver enthalten üblicherweise eine in Wasser lösliche oder zumindest quellbare Pudergrundlage, beispielsweise Stärke, Maltodextrin, Cyclodextrin, Silicagel, Polyethylenglykole, etc., vorzugsweise in einer Menge von etwa 10 bis etwa 70, insbesondere etwa 20 bis etwa 50 Gew.-% der Gesamtzusammensetzung.

Diese Bestandteile sind ebenso bekannt wie die Mitverwendung von Tensiden, vorzugsweise in einer Menge von etwa 5 bis 30 Gew.-% des Pulvers.

Geeignete Tenside sind anionische Tenside wie langkettige N-Acylaminocarbonsäuren und deren Salze wie N-Lauroylsarkosinat und -glutamat, amphotere Tenside wie Betaine, z. B. Cocoamidopropylbetain, sowie nichtionische und kationische Tenside, z. B. C₁₂-C₁₈-Alkylpolyglykolether, und Ethylenoxid/ Propylenoxid-Copolykondensate und langkettige quaternäre Ammoniumverbindungen, z. B. Distearyl- oder Dilauryldimethylammoniumchlorid.

Die erfindungsgemäßen Farbpulver können die in solchen Zusammensetzungen üblichen Zusätze enthalten, deren Art und Charakter von der Applikationsform des Mittels abhängig ist, z. B. Fette, Fettalkohole, Emulgatoren, pH-Regulatoren, Eiweißhydrolysate, Lösungsvermittler, Konservierungsmittel, Parfums, etc.

Die erfindungsgemäßen Farbpulver werden vor ihrer Anwendung mit Wasser im Verhältnis von etwa 1 : 5 bis etwa 1 : 30, vorzugsweise 1 : 10 bis 1 : 20, angerührt, auf dem Haar gleichmäßig verteilt und nach etwa 20- bis 30-minütiger Einwirkung durch Shampoonieren entfernt.

Die folgenden Beispiele beschreiben die Zusammensetzung der erfindungsgemäßen Mittel und zeigen ihre Überlegenheit gegenüber konventionellen Produkten.

Eine Rahmenrezeptur für ein erfindungsgemäßes Farbstoffpulver läßt sich etwa folgendermaßen darstellen:

| | |
|---|---|
| Pulvergrundlage, z. B. Stärke, Kieselgur, Cyclodextrin, Polyethylenglykol 1200 - 2000 | etwa 10 bis 70, insbesondere 20 bis 50 Gew.-% |
| Hilfs- und Pflegestoffe, z. B. Honigtrockenextrakt, Eiweißhydrolysate, oberflächenaktive Substanzen, kationaktive oder anionaktive Polymere | etwa 1 bis 20, insbesondere 5 bis 15 Gew.-% |
| Direktziehende Farbstoffe, je nach gewünschter Färbung | etwa 0,1 bis 25, insbesondere 0,5 bis 10 Gew.-% |
| Konservierungsmittel, Parfum | q.s. |

Die folgenden Einzelbeispiele erläutern beispielhaft einige mit der Erfindung erzielbare Farbtöne:

| **Beispiel Nr.** | **1** | **2** | **3** |
|---|---|---|---|
| Hydroxypropyl-Guar | 14,00 | | |
| Hydroxypropyl-Guartrimoniumchlorid | | 14,00 | 14,00 |
| Cyclodextrin | 3,50 | 3,60 | 3,30 |
| PEG-1500 | 20,00 | 19,50 | 20,00 |
| Fettalkoholpolyglykolether (6 EO) | 7,00 | 6,50 | 6,60 |
| Fettalkoholpolyglykolether (50 EO) | 6,00 | | 6,50 |
| Ethylen-/Propylenoxid-Kondensat (Poloxamer) | | 6,50 | |
| Weizenproteinhydrolysat | 2,40 | 2,50 | 2,30 |
| Honigtrockenextrakt | 3,00 | 3,30 | 3,30 |
| Stärke | 38,00 | 38,00 | 38,00 |
| Kaliumsorbat | 3,30 | 3,30 | 3,30 |
| C.I.-Nr. 56,059 | 0,65 | 0,65 | |
| C.I.-Nr. 12,250 | 2,00 | 0,15 | |
| C.I.-Nr. 12,251 | 0,15 | 2,00 | |
| C.I.-Nr. 75,480 | | | 2,70 |
| **Erzielter Farbton** | **Hellblond** | **Hellblond** | **Kupferblond** |

Die Anwendung der in den Beispielen beschriebenen Zusammensetzungen erfolgte durch Anrühren von Jeweils 5 g Pulver mit 80 g Wasser, wobei jeweils homogene, nicht klumpende Zusammensetzungen erhalten wurden, 20-minütiger Einwirkung bei Raumtemperatur und anschließendes Ausspülen mit Wasser sowie Trocknen.

Es wurden glänzende, dauerhafte Färbungen erhalten.

### Vergleichsversuch

Es wurden 3 Farbzusammensetzungen hergestellt, wovon die Zusammensetzung 3a der im vorstehenden Beispiel 3 beschriebenen Zusammensetzung entsprach.

Beispiel 3b enthielt statt des Hydroxypropyl-Guartrimo-niumchlorids die gleiche Menge Hydroxypropyl-Guar; Beispiel 3c (Vergleichsbeispiel) die gleiche Menge Hydroxyethylcellulose.

Je 5 g Farbpulver wurden mit 80 g Wasser angerührt und einmal auf Woll-Läppchen und zum anderen auf gebleichte Strähnen Menschenhaar aufgetragen und nach 20-minütiger Einwirkung mit Wasser gewaschen, getrocknet und die Farbunterschiede durch die bekannte Helligkeitsmessung mittels des Chrom-Meters CR-200 (Minolta) gegen ungefärbte Referenzmuster bestimmt.

Je höher der Wert für ΔE, desto farbintensiver ist die Färbung.

Die erzielten Ergebnisse sind in den folgenden Tabellen wiedergegeben.

**Tabelle 1**

| **Woll-Läppchen** | | **L** | **a** | **b** | **ΔE** |
|---|---|---|---|---|---|
| unbehandeltes Refernzmuster | | 87,13 | -0,95 | 10,78 | |
| Proben | 3a | 67,08 | 24,94 | 46,27 | 48,29 |
| nach den | 3b | 67,88 | 24,51 | 45,54 | 47,19 |
| Beispielen | 3c | 68,33 | 23,33 | 43,10 | 44,58 |

**Tabelle 2**

| **Gebleichte Haarsträhnen** | | **L** | **a** | **b** | **ΔE** |
|---|---|---|---|---|---|
| unbehandeltes Refernzmuster | | 72,28 | 2,81 | 4,34 | |
| Proben | 3a | 57,23 | 19,19 | 34,89 | 37,79 |
| nach den | 3b | 58,25 | 19,29 | 34,65 | 37,24 |
| Beispielen | 3c | 58,95 | 18,08 | 32,33 | 34,56 |

Die Ergebnisse zeigen klar den farbverstärkenden Effekt der Guar Gum Derivate.

## Patentansprüche

1. Pulverförmiges Mittel zum Färben und Tönen von menschlichen Haaren, enthaltend mindestens einen direktziehenden Farbstoff und eine pulverförmige Grundlage, dadurch gekennzeichnet, daß es 1 bis 50 Gew.-%, berechnet auf die Gesamtzusammensetzung des Farbpulvers, mindestens eines Hydroxy-C₂-C₄-alkylGuar Gums bzw. dessen quaternären Salzen enthält.

2. Mittel nach Anspruch 1, dadurch gekennzeichnet, daß es 2,5 bis 35 Gew.-%, berechnet auf die Gesamtzusammensetzung des Farbpulvers, mindestens eines Hydroxy-C₂-C₄-alkyl-Guar Gums bzw. dessen quaternären Salzen enthält.

3. Mittel nach Anspruch 2, dadurch gekennzeichnet, daß es als Hydroxyalkyl-Guar Gum Hydroxypropyl-Guar Gum bzw. ein quaternäres Salz desselben enthält.

4. Mittel nach einem oder mehreren der Ansprüche 1 bis 3, dadurch gekennzeichnet, daß es als direktziehenden Farbstoff mindestens einen kationischen Farbstoff enthält.

## Claims

1. Composition for dyeing of human hair, comprising at least one direct dye and a pulverulent carrier material, containing from 1% to 50% by wt., calculated to the total composition of the dyeing powder, of at least one hydroxy-C₂-C₄-alkyl Guar gum or the quaternary salts thereof.

2. Composition according to claim 1, characterized in that it contains 2.5% to 35% by wt., calculated to the total composition of the dyeing powder, of at least one hydroxy-C₂-C₄-alkyl Guar gum or the quaternary salts thereof.

3. Composition according to claim 2, characterized in that it contains as hydroxyalkyl Guar gum hydroxypropyl Guar gum or a quaternary salt thereof.

4. Composition according to one or more of claims 1 to 3, characterized in that it contains at least one cationic dyestuff as direct dye.

## Revendications

1. Agent pulvérulent pour le colorer et nuancer les cheveux humains, contenant au moins un colorant direct et une base pulvérulente, caractérisé en ce qu'il contient de 1 à 50% en poids par rapport à la composition totale de la poudre colorante, d'au moins une gomme de guar hydroxyalkylée en C₂ à C₄ ou ses sels quaternaires.

2. Agent selon la revendication 1, caractérisé en ce qu'il contient de 2,5 à 35% en poids par rapport à la composition totale de la poudre colorante, d'au moins une gomme de guar hydroxyalkylée en C₂ à C₄ ou ses sels quaternaires.

3. Agent selon la revendication 2, caractérisé en ce qu'il contient, en tant que gomme de guar hydroxyalkylée, une gomme de guar hydroxypropylée ou ses sels quaternaires.

4. Agent selon l'une ou plusieurs des revendications 1 à 3, caractérisé en ce qu'il contient, en tant que colorant direct, au moins un colorant cationique.
